# EUROPÄISCHE PATENTSCHRIFT

(11) **EP 1 912 739 B1**
(45) Veröffentlichungstag und Bekanntmachung des Hinweises auf die Patenterteilung: **11.10.2017**
(21) Anmeldenummer: 06762915.4
(22) Anmeldetag: 01.08.2006
(51) Int. Cl.: B01J 23/52, B01J 23/66, C07C 51/235, B01J 37/02, B01J 35/10

(54) **GETRÄGERTER GOLDKATALYSATOR**
SUPPORTED GOLD CATALYST
CATALYSEUR SUPPORTE A BASE D'OR

(30) Priorität: 05.08.2005 DE 102005036890
(43) Veröffentlichungstag der Anmeldung: 23.04.2008
(73) Patentinhaber: Südzucker Aktiengesellschaft Mannheim/Ochsenfurt, 68165 Mannheim (DE)
(72) Erfinder: HAJI BEGLI, Alireza, 67305 Ramsen (DE); BAATZ, Christine, 67551 Worms (DE); DECKER, Nadine, 38106 Braunschweig (DE); PRÜSSE, Ulf, 38118 Braunschweig (DE); VORLOP, Klaus-Dieter, 38102 Braunschweig (DE)
(74) Vertreter: Schwahn, Hartmut
(86) Internationale Anmeldenummer: PCT/EP2006/007584
(87) Internationale Veröffentlichungsnummer: WO 2007/017157

(56) Entgegenhaltungen:
- WO-A-03/068389
- DE-A1- 10 319 917
- GB-A- 2 408 956
- PRATI L ET AL: "Gold on Carbon as a New Catalyst for Selective Liquid Phase Oxidation of Diols" JOURNAL OF CATALYSIS, ACADEMIC PRESS, DULUTH, MN, US, Bd. 176, Nr. 2, 10. Juni 1998 (1998-06-10), Seiten 552-560, XP004447366 ISSN: 0021-9517
- BERNDT H ET AL: "Oxygen adsorption on Au/Al2O3 catalysts and relation to the catalytic oxidation of ethylene glycol to glycolic acid" APPLIED CATALYSIS A: GENERAL, ELSEVIER SCIENCE, AMSTERDAM, NL, Bd. 244, Nr. 1, 8. Mai 2003 (2003-05-08), Seiten 169-179, XP004421324 ISSN: 0926-860X

## Beschreibung

Die Erfindung betrifft Verfahren zur Herstellung von geträgerten Goldkatalysatoren aus porösem Metalloxidträger und Chlorogoldsäure-Precursor, wobei der Träger mit wässriger Lösung des Chlorogoldsäure-Precursors in Kontakt gebracht wird. Die Erfindung betrifft auch einen Metalloxid-geträgerten Goldkatalysator und seine Verwendung zur Oxidation von Alkoholen, Aldehyden, Polyhydroxyverbindungen und Kohlenhydraten.

Es besteht regelmäßig Bedarf an hochaktiven und stabilen Katalysatoren, die vor allem für die Oxidation von organischen Verbindungen wie Alkoholen, Aldehyden, Polyhydroxyverbindungen und Mono-, Oligo- und Polysacchariden eingesetzt werden können.

Bekannt ist die Verwendung geträgerter Palladium- und PlatinKatalysatoren zur Oxidation von Glucose. Diese ist aber aufgrund der geringen Selektivität und Umsetzungsrate erheblich eingeschränkt. Auch kommt es zu einer relativ schnellen Deaktivierung der Katalysatoren durch Blockierung der KatalysatorOberfläche aufgrund von Adsorption und/oder aufgrund von Vergiftungseffekten.

Zur großtechnischen Produktion von Oxidationsprodukten aus Kohlenhydraten müssen daher nach wie vor Fermentationsprozesse eingesetzt werden, die mit hohem apparativem Aufwand und mit Abwasserbelastungen einhergehen.

Aus diesem Grund sollen neue Katalysator-Typen entwickelt werden, die eine effektive katalytische Oxidation, besonders von Kohlenhydraten, beispielsweise zur Herstellung von Aldonsäuren unter Verwendung von Disauerstoff als Oxidationsmittel, ermöglichen und neben hoher Aktivität und Selektivität eine lange Lebensdauer aufweisen.

Geträgerte Goldkatalysatoren sind ebenfalls bekannt. Sie werden hauptsächlich zur Oxidation von CO oder Propen in der Gasphase und für Selektivhydrierungen eingesetzt. Kohlenstoff-geträgerte Goldkatalysatoren können auch zur selektiven Oxidation von D-Glucose zu D-Gluconsäure in der Flüssigphase eingesetzt werden. Aus der DE 10319917 A1 sind auch geträgerte Goldkatalysatoren mit nanodispers verteilten Goldpartikeln auf Kohlenstoff- oder Metalloxid-Trägern bekannt. Diese werden vor allem zur C1-selektiven Oxidation von Glucose und anderen Kohlenhydraten eingesetzt. Die Aktivität dieser Katalysatoren ist jedoch nicht befriedigend.

Verfahren zur Herstellung von Goldkatalysatoren durch Imprägnierung des Trägers nach der "Incipient-Wetness-Methode" sind bekannt. GB 2 408 956 A, Prati L. et al. Journal of Catalysis 176(2), 1998: 552-560, Berndt H. et al. Applied Catalysis A 244(1), 2003:169-179, WO 03/068389 A1 und Zanella et al. Journal of Physical Chemistry B 106, 2002:7634-7642, beschreiben die Herstellung von geträgerten Goldkatalysatoren mittels Incipient-Wetness-Imprägnierung der Träger mit einer Precursorlösung aus Chlorogoldsäure gelöst in Wasser.

In der Literatur werden solche Imprägnierungsmethoden aber als vergleichbar wenig geeignet für die Synthese hoch aktiver Goldkatalysatoren beschrieben. Das liegt daran, dass bei diesen Verfahren in der Regel nur große Goldpartikel (größer als 10 nm) erhalten werden.

Das der vorliegenden Erfindung zugrunde liegende technische Problem besteht daher im Wesentlichen darin, verbesserte Metalloxid-geträgerte Goldkatalysatoren und Verfahren zu deren Herstellung bereitzustellen, die eine verbesserte Aktivität, vor allem bei der Oxidation von organischen Verbindungen wie Alkoholen, Aldehyden und Polyhydroxyverbindungen, aufweisen.

Weiter liegt der Erfindung das Problem zugrunde, ein Verfahren zur selektiven und effektiven Oxidation von Kohlenhydraten, insbesondere zur Herstellung von Aldonsäuren, bereitzustellen, das die Nachteile des Standes der Technik überwindet.

Das zugrunde liegende technische Problem wird gelöst durch die Bereitstellung eines Verfahrens mit den Merkmalen des Anspruchs 1 zur Herstellung eines geträgerten Goldkatalysators aus porösem Metalloxidträger und Chlorogoldsäure-Precursor, wobei in einem Schritt a) der Metalloxidträger bereitgestellt wird, in einem Schritt b) der Träger mit einer Lösung des Chlorogoldsäure-Precursors in Kontakt gebracht wird. In Schritt b) wird daraus ein imprägnierter Katalysator-Vorläufer erhalten, welcher in einem nachfolgenden Schritt c) getrocknet wird. Das erfindungsgemäße Verfahren ist besonders dadurch gekennzeichnet, dass in Schritt a) der Träger in trockener und bevorzugt pulverisierter oder granulierter Form oder als Formkörper bereitgestellt wird und dass in Schritt b) das Volumen der Lösung des Chlorogoldsäure-Precursors maximal so groß gewählt wird, dass es dem Porenvolumen des Trägers entspricht. Es kann kleiner gewählt werden, nicht aber größer als das Porenvolumen.

Ist das spezifische Porenvolumen des Trägers nicht bekannt, wird vorzugsweise das dem trockenen Träger zugeführte Volumen der Precursorlösung empirisch bestimmt, indem die Precursorlösung schrittweise zu dem trockenen Träger gegeben wird, solange bis der Träger kein weiteres Volumen der Precursorlösung mehr aufnehmen kann. Dies wird vor allem an dem einsetzenden feuchten Erscheinungsbild des Trägermaterials erkennbar. Es ergibt sich für jede Metalloxidträger-Sorte eine spezifische Aufnahmefähigkeit [in MI/g_{Katalysatorträger}], die vor allem vom Oberfläche/Volumen-Verhältnis, von der Porengröße und vom Trocknungsgrad des Metalloxidträgers abhängt. Unter "trocken" wird verstanden, dass der poröse Metalloxidträger im Wesentlichen keine Feuchtigkeit im Porenvolumen enthält, sodass in das Porenvolumen Precursorlösung aufgenommen werden kann.

In einer besonders bevorzugten Variante werden die Schritte a) bis c) mehrmals, das heißt mindestens zweimal, hintereinander durchgeführt. In einer alternativen Variante werden die Schritte b) und c) gleichzeitig, das heißt parallel nebeneinander, durchgeführt.

In einer bevorzugten Ausführungsform findet in Schritt b) das Inkontaktbringen des Metalloxidträgers mit dem Chlorogoldsäure-Precursors durch Zutropfen des Precursors zum Träger unter Verrühren statt. In einer bevorzugten Variante wird der Precursor auf den Träger aufgesprüht, wobei der Träger bevorzugt verrührt wird.

Vorzugsweise wird während des Verrührens der Träger mit dem aufgebrachten Precursor getrocknet (Schritt c)). In einer Variante findet das Inkontaktbringen des Precursors mit dem Träger in einem Dragierkessel beziehungsweise einem Pelletierteller statt, wobei bevorzugt zugetropft oder aufgesprüht wird und gegebenenfalls gleichzeitig getrocknet wird. In einer weiteren Variante befindet sich der Träger in einer Wirbelschicht und der Precursor wird in die Wirbelschicht eingebracht, bevorzugt eingesprüht; dabei wird der Träger vorzugsweise mit dem aufgebrachten Precursor getrocknet (Schritt c)).

Erfindungsgemäß wird als Lösung des Chlorogoldsäure-Precursors eine Lösung von Tetrachlorogoldsäure (HAuCl₄) in wässriger Säure, insbesondere Salzsäure, eingesetzt, wobei die Konzentration der Säure von 0,1 mol/l bis 12 mol/l, bevorzugt von 1 mol/l bis 4 mol/l, besonders bevorzugt 2 mol/l, beträgt. Dabei beträgt der pH-Wert der fertigen Precursorlösung stets 1 oder weniger. Vorzugsweise oder gegebenenfalls - je nach Anwendungsfall - enthält die erfindungsgemäß verwendete Precursorlösung noch mindestens eine weitere Säure. Selbstverständlich können anstelle der Salzsäure weitere anorganische oder organische Säuren eingesetzt werden.

Besonders bevorzugt wird zur Herstellung der wässrigen Precursorlösung die erforderliche Menge an Tetrachlorogoldsäure direkt in die wässrige Säure eingewogen und gelöst. Erfindungsgemäß wird zum Lösen der Tetrachlorogoldsäure wässrige Salzsäure in einer Konzentration von 0,1 mol/l bis 12 mol/l, besonders von 1 mol/l bis 4 mol/l und besonders bevorzugt von 2 mol/l eingesetzt.

TEM-Messungen haben gezeigt, dass die erfindungsgemäß hergestellten Katalysatoren überraschenderweise sehr kleine und aktive Partikelgrößen von weniger als 10 nm, insbesondere von 1 nm bis 10 nm, vorzugsweise von 1 nm bis 9 nm, besonders von 1 nm bis 5 nm oder sogar von 1 nm bis 2 nm, aufweisen. Es ist den Erfindern mit dem erfindungsgemäßen Verfahren erstmals gelungen, nach der "incipient wetness"-Methode katalytisch aktive Goldpartikel in Größen von deutlich unter 10 nm zu präparieren. Diese Erkenntnisse sind überraschend und stehen den Beschreibungen der einschlägigen Literatur entgegen. Die erhaltenen Goldkatalysatoren zeigen eine bisher nicht erreichte Aktivität, beispielsweise bei der Umsetzung von Glucose. Besonders durch Verwendung einer stark sauren Precursorlösung (zum Beispiel 2 mol/l HCl als Lösungsmittel für Tetrachlorogoldsäure) und bei Verwendung von porösem Aluminiumoxid wie Puralox KR-90 als Trägermaterial gelang die Präparation des bislang aktivsten Goldkatalysators für die Glucoseoxidation. Ein erfindungsgemäß hergestellter Katalysator weist eine Aktivität von bis zu 2200 mmol g_{Metall}⁻¹ min⁻¹ auf. Dies ist eine Steigerung gegenüber den im Stand der Technik bekannten Goldkatalysatoren etwa um das Vierfache.

HAuCl₄ ist in wässriger Lösung nicht stabil sondern wird hydrolysiert. Es findet in mehreren hintereinander geschalteten Gleichgewichten ein sukzessiver Austausch des Chlorids gegen Wasser und Hydroxygruppen statt: [AuCl₄]⁻, [AuCl₃(OH)]⁻, [AuCl₂(OH)₂]⁻, [AuCl₂(OH)], [AuCl(OH)₂], [Au(OH)₃], [Au(OH)₄]⁻. Diese Gleichgewichte sind zeit- und pH-abhängig. Durch einen hinreichend niedrigen pH-Wert kann die Hydrolyse verhindert werden.

Ohne an die Theorie gebunden zu sein, dominiert in stark saurer wässriger Lösung (2 mol/l HCl) der Tetrachlorokomplex [AuCl₄]⁻. Die Gegenwart dieses Komplexes führt überraschend dazu, dass vor allem bei der Reduktion des Katalysatorvorläufers sehr kleine Partikel stabilisiert werden. In anderen, schwächer sauren Lösungen kommt es wahrscheinlich nach und nach zu einem sukzessiven Austausch der Chloridionen gegen Wasser und Hydroxidionen.

Bevorzugt werden als Träger Metalloxide wie Aluminiumoxid, Titandioxid oder Siliziumoxid eingesetzt. Besonders bevorzugt wird Aluminumoxid (Al₂O₃), beispielsweise Puralox SCFa-90 oder Puralox KR-90, verwendet.

Vorzugsweise wird in Schritt c) bei Temperaturen von größer oder gleich Raumtemperatur, bevorzugt von 60 °C bis 200 °C, besonders bevorzugt von 60 °C bis 100 °C getrocknet.

Bevorzugt wird in einem weiteren Schritt d), der bevorzugt nach dem Schritt c) durchgeführt wird, der Katalysatorvorläufer reduziert. Dies geschieht bevorzugt im Wasserstoffstrom. Vorzugsweise hat der Wasserstoffstrom einen Wasserstoffgehalt von 5 Vol.-% bis 15 Vol.-%, bevorzugt 10 Vol.-%. Je nach Anwendungsgebiet kann der Wasserstoffstrom gegebenenfalls mindestens ein Inertgas wie Stickstoff oder Edelgas, enthalten. Besonders bevorzugt besteht der Wasserstoffstrom aus Wasserstoffgas und mindestens einem Inertgas. Alternativ kann die Reduktion als Flüssigphasenreduktion in an sich bekannter Weise mit geeigneten Reduktionsmitteln wie Natriumborhydrid, Formiatsalzen, Kohlenhydraten, Formaldehyd oder Hydrazin erfolgen.

Werden in einer bevorzugten Ausführungsform des erfindungsgemäßen Verfahrens die Schritte a) bis c), besonders die Schritte b) und c), mehrmals hintereinander durchgeführt, ist bevorzugt vorgesehen, dass zwischenzeitlich, vorzugsweise nach jedem Durchlaufen der Schritte a) bis c), besonders b) und c), der Katalysator-Vorläufer reduziert wird (Schritt d)).

Bevorzugt wird die Reduktion in Schritt d) bei Temperaturen von größer oder gleich 250 °C durchgeführt. Erfindungsgemäß bevorzugt erfolgt die Reduktion von 10 Minuten bis 300 Minuten, vorzugsweise von 80 bis 120 Minuten.

Erfindungsgemäß ist auch vorgesehen, dass zu dem Träger und/oder der wässrigen Lösung des Chlorogoldsäure-Precursors mindestens ein Dotierungszusatz zugegeben werden kann. Dieser wird bevorzugt ausgewählt aus Oxiden der Alkalimetalle, der Erdalkalimetalle und der Seltenerdmetalle. Besonders bevorzugt sind Dotierungen mit Natrium, Kalium, Caesium, Calcium, Cer und/oder Samarium. Bevorzugt wird der mindestens eine Dotierungszusatz in einem Anteil von 0,01 Gew.-% bis 1 Gew.-% zugegeben.

Ein weiterer Gegenstand der vorliegenden Erfindung ist demgemäß auch die Verwendung eines Chlorogoldsäure-Precursors der eine Lösung oder Suspension von Tetrachlorogoldsäure (HAuCl₄) in einem Lösungsmittel enthält, beziehungsweise daraus besteht, wobei das Lösungsmittel wässrige Säure in einer Konzentration von 0,1 mol/l bis 12 mol/l, bevorzugt von 1 mol/l bis 4 mol/l, besonders bevorzugt von 2 mol/l, ist. Erfindungsgemäß ist die Säure Salzsäure (HCl). dDie Salzsäure liegt gegebenenfalls in Verbindung mit mindestens einer weiteren Säure vor. Erfindungsgemäß wird dieser Chorogoldsäure-Precursor zur Herstellung eines Metalloxid-geträgerten Goldkatalysators mittels der vorstehend beschriebenen Inicpient-Wetness-Imprägnierung, eingesetzt.

Weiterer Gegenstand der vorliegenden Erfindung ist auch ein Metalloxid-geträgerter Goldkatalysator, der nach dem vorgenannten erfindungsgemäßen Verfahren herstellbar ist beziehungsweise hergestellt wird. Der erfindungsgemäße Katalysator ist besonders dadurch gekennzeichnet, dass die mittlere Größe der Goldpartikel auf dem Träger im Wesentlichen kleiner als 10 nm, bevorzugt kleiner oder gleich 5 nm, besonders bevorzugt von 1 nm bis 2 nm ist.

Vorzugsweise weist der erfindungsgemäße Katalysator einen Goldanteil von 0,01 Gew.-% bis 10 Gew.-%, bevorzugt von 0,01 Gew.-% bis 2 Gew.-%, besonders bevorzugt von 0,3 Gew.-% auf.

Schließlich ist ein weiterer Gegenstand der vorliegenden Erfindung die Verwendung des vorgenannten erfindungsgemäßen Katalysators zur Oxidation organischer Edukte, die insbesondere ausgewählt sind aus Alkoholen, Aldehyden und Polyhydroxyverbindungen. Erfindungsgemäß bevorzugt wird der Katalysator in einer heterogenen Katalyse eingesetzt. Das heißt, der Katalysator liegt als Feststoff vor, während die zu oxidierenden Edukte in fluider Phase, beispielsweise als wässrige Lösung, vorliegen. Der bevorzugt zur Oxidation eingesetzte Disauerstoff wird dann als Gas durch die flüssige Phase hindurchgeperlt und durch intensives Rühren in der Flüssigphase verteilt und gelöst. Der Katalysator wird vorzugsweise in Form eines Pulvers oder Granulats eingesetzt. In einer weiteren bevorzugten Variante werden Formkörper, beispielsweise Zylinder, Hohlzylinder, Kugeln oder Stränge eingesetzt.

In bevorzugter Ausführungsform wird eine wässrige Lösung des zu oxidierenden Edukts oder Eduktgemischs hergestellt, die das Edukt mindestens etwa 10 mmol/l, bevorzugt mindestens etwa 100 mmol/l, 150 mmol/l, 200 mmol/l, 250 mmol/l, 1000 mmol/l oder 1500 mmol/l enthält. Anschließend wird der wässrigen Edukt-Lösung der vorzugsweise pulverförmige erfindungsgemäße Katalysator in einer Menge von etwa 10 mg/l bis 10 g/l zugesetzt, wobei vorzugsweise pro Liter etwa 1 g Katalysator eingesetzt werden. Vorzugsweise beträgt das Verhältnis zwischen der Menge des/der zu oxidierenden Edukts und der Menge des auf dem Metalloxid-Träger enthaltenen Goldes mindestens etwa 300 - 400.000, bevorzugt mindestens 300, 500, 1.000, 2.000, 4.000, 10.000, 20.000, 50 000, 100.000, 200.000 oder 400.000.

Vorzugsweise wird die Oxidation des Edukts oder Eduktgemischs bei einem pH-Wert von 7 bis 11, vorzugsweise von 8 bis 10, durchgeführt. Vorzugsweise wird eine Temperatur von 20 °C bis 140 °C, von 40 °C bis 90 °C und besonders bevorzugt von 40 °C bis 80 °C eingesetzt. Der Druck beträgt vorzugsweise etwa 1 bar bis etwa 25 bar. Vorzugsweise wird Sauerstoff und/oder Luft mit einer Begasungsrate von 100 ml/(min x L_{Reaktorvolumen}) bis 10000 ml/(min x L_{Reaktorvolumen}), vorzugsweise von 500 ml/(min x L_{Reaktorvolumen}), durch die wässrige Eduktlösung des Edukts, des Gemisches oder der Zusammensetzung hindurchgeperlt.

Es zeigt sich, dass bei den erfindungsgemäßen Goldkatalysatoren bei der Oxidation von Aldosen eine 100% Selektivität für die Aldehydposition auftritt. Die erfindungsgemäßen Goldkatalysatoren eignen sich daher außerdem für die selektive Oxidation von Kohlenhydraten. Darunter wird insbesondere die Oxidation einer oxidierbaren Aldehyd-Gruppe am C1-Kohlenstoffes eines Kohlenhydrats zu einer Carboxyl-Gruppe verstanden, wohingegen Alkohol-Gruppen an anderen Kohlenstoffatomen des Kohlenhydrates nicht oxidiert werden. Im Ergebnis wird daher vorzugsweise Aldonsäure erhalten. Bei den erfindungsgemäß bevorzugt eingesetzten Kohlenhydraten handelt es sich vorzugsweise um Aldosen, die am C1-Kohlenstoff eine oxidierbare Aldehyd-Gruppe aufweisen, oder um 2-Ketosen, bei denen am C1-Kohlenstoffatom eine oxidierbare Aldehyd-Gruppe eingeführt werden kann. Durch die selektive Oxidation der Aldehyd-Gruppe einer Aldose wird eine Aldonsäure erhalten. Bei der selektiven Oxidation eines Gemisches von Aldosen wird daher ein Gemisch unterschiedlicher Aldonsäuren erhalten.

Die vorliegende Erfindung betrifft daher auch die Verwendung der erfindungsgemäßen Katalysatoren zur Herstellung einer Aldonsäure oder eines Gemisches verschiedener Aldonsäuren durch selektive Oxidation einer oder mehrerer Aldosen mit einer oxidierbaren Aldehyd-Gruppe.

Die vorliegende Erfindung betrifft daher auch die Verwendung zur Herstellung einer Aldonsäure oder eines Gemisches verschiedener Aldonsäuren unter Verwendung einer oder mehrerer 2-Ketosen, wobei die 2-Ketose(n) zunächst in die tautomere(n) Aldose-Form(en) mit einer oxidierbaren Aldehyd-Gruppe überführt und dann unter Verwendung des Katalysators selektiv oxidiert wird/werden.

Erfindungsgemäß umfassen die zu oxidierenden Kohlenhydrate sowohl monomere Polyhydroxyaldehyde oder Polyhydroxyketone, also Monosaccharide, deren Dimere bis Dekamere, das heißt Oligosaccharide wie Disaccharide, Trisaccharide etc., und die makromolekularen Polysaccharide. Unter "Monosacchariden" werden im Zusammenhang mit der vorliegenden Erfindung Verbindungen der allgemeinen chemischen Formel CₙH₂ₙOₙ mit 3 bis 7 Sauerstoff-Funktionen verstanden, wobei natürliche Monosaccharide im Wesentlichen Hexosen und Pentosen sind. Die Kohlenstoff-Kette eines Monosaccharids kann unverzweigt oder verzweigt sein. Unter "Oligosaccharide" werden Verbindungen verstanden, die durch Vereinigung von 2 bis 10 Monosaccharid-Moleküle unter Wasseraustritt erhalten werden.

Besonders bevorzugt wird der Katalysator zur selektiven Oxidation von Kohlenhydraten, ausgewählt aus Monosacchariden wie Glucose, Galaktose, Mannose, Xylose und Ribose sowie Disaccharid-Aldosen wie Maltose, Laktose, Cellobiose und Isomaltose sowie Disaccharid-2-Ketosen wie Palatinose sowie Stärkesirup und Maltodextrinen sowie Gemischen dieser Kohlenhydrate eingesetzt.

Bei der Oxidation von Glucose wird unter Verwendung des erfindungsgemäßen Verfahrens Gluconsäure als Oxidationsprodukt erhalten. Bei der Oxidation von Galaktose wird unter Verwendung des erfindungsgemäßen Verfahrens Galaktonsäure als Oxidationsprodukt erhalten.

In einer weiteren bevorzugten Ausführungsform ist das zu oxidierende Kohlenhydrat ein Oligosaccharid, insbesondere ein Disaccharid. Das zu oxidierende Disaccharid ist vorzugsweise eine Disaccharid-Aldose wie Maltose, Laktose, Cellobiose oder Isomaltose. Erfindungsgemäß wird bei der selektiven Oxidation von Maltose unter Verwendung des erfindungsgemäßen Verfahrens Maltobionsäure als Oxidationsprodukt erhalten. Unter Verwendung des erfindungsgemäßen Verfahrens wird bei der Laktose-Oxidation Lactobionsäure als Oxidationsprodukt erhalten.

In einer weiteren bevorzugten Ausführungsform der Erfindung ist das zu oxidierende Oligosaccharid eine Disaccharid-Ketose. Bei der zu oxidierenden Disaccharid-Ketose handelt es sich vorzugsweise um Palatinose (Isomaltulose). Vor der Oxidation wird Palatinose erfindungsgemäß in die tautomere Aldose-Form überführt, die dann oxidiert wird.

In einer weiteren bevorzugten Ausführungsform der Erfindung ist das zu oxidierende Kohlenhydrat ein Maltodextrin. Maltodextrine sind durch den enzymatischen Stärkeabbau gewonnene wasserlösliche Kohlenhydrate, insbesondere Dextrose-Äquivalente, mit einer Kettenlänge von 2 bis 30, vorzugsweise 5 bis 20 Anhydroglucose-Einheiten und einem Anteil an Maltose. Bei der selektiven Oxidation von Maltodextrin wird unter Verwendung des erfindungsgemäßen Verfahrens ein Oxidationsprodukt erhalten, das erfindungsgemäß entsprechend der Zusammensetzung neben den Oligosaccharid-Aldonsäuren einen Anteil von Maltobionsäure und Gluconsäure aufweist.

In einer weiteren bevorzugten Ausführungsform ist das zu oxidierende Kohlenhydrat ein Stärkesirup. Unter einem Stärkesirup wird ein Glucosesirup verstanden, der aus Stärke gewonnen wird und vor allem als gereinigte wässrige Lösung vorliegt, wobei die Trockenmasse in der Regel mindestens 70 % beträgt.

In einer weiteren bevorzugten Ausführungsform ist das zu oxidierende Kohlenhydrat ein Furfural. Das zu oxidierende Furfural ist vorzugsweise Hydroxymethylfurfural (HMF) oder Glycosyloxymethylfurfural (GMF).

Die Erfindung wird durch die nachfolgenden Beispiele und Figuren näher erläutert, wobei die Beispiele nicht beschränkend zu verstehen sind.
Figur 1 zeigt die TPR-Profile der Katalysatoren.
Figur 2 zeigt Abhängigkeit der Aktivität der hergestellten Katalysatoren von der verwendeten Precursorlösung.
Figur 3 zeigt die Langzeitstabilität des Katalysators über 20 "repeated batch"-Versuche.
Figur 4 zeigt den Einfluss unterschiedlicher Promotoren auf die max. spez. Aktivität der erfindungsgemäß hergestellten Goldkatalysatoren.

### Beispiel 1: Katalysatorherstellung

### Trägermaterial

Als Trägermaterial wurden beispielhaft die Aluminiumoxide Puralox KR-90 und Puralox SCFa-90, eine NaO dotierte Sonderform, (beide Fa. Sasol) verwendet. Diese weisen folgende Parameter auf:

**Tabelle 1**

| | | Puralox SCFa-90, NaO dotiert | Puralox KR-90 |
|---|---|---|---|
| Phase | | γ/δ- Phase | δ/τ- Phase |
| BET-Oberfläche: | | 90-100 m²/g | 90 m²/g |
| Kornverteilung: | < 25 µm | 26,3 % | 26,8 % |
| | < 45 µm | 55,8 % | 54,0 % |
| | < 90 µm | 99,7 % | 95,6 % |
| Porenvolumen: | | ca. 0,5 ml/g | ca. 0,8 ml/g |
| Schüttdichte: | | 0,6 g/ml | 0,3 g/ml |
| Na-Gehalt: | | 0,25 - 0,3 % | 0,03 % |

### Herstellung des Chlorogoldsäure-Precursors

Die benötigte Menge Tetrachlorogoldsäure in kristalliner Form (Fa. Chempur (50 % Au)) wird im dem Volumen eines Lösungsmittels gelöst, dass maximal dem Porenvolumen der verwendeten Trägermenge entspricht.

Es wurden verschiedene Katalysatoren hergestellt, bei denen der Precursor HAuCl₄ in Salzsäure, Wasser und Kalilauge gelöst wurde. Außerdem wurde eine längere Zeit gelagerte, wässrige Lösung des Precursors (25 g/l Au) mit Wasser und Salzsäure entsprechend verdünnt. Folgende Ansätze an Chlorogoldsäure-Precursor wurden hergestellt:
1. Precursor in 2 mol/l HCl eingewogen und gelöst (erfindungsgemäß)
2. Precursorlösung mit 0,2 mol/l HCl verdünnt aus wässriger Precursor-Stammlösung (Vergleichsbeispiel)
3. Precursorlösung in Wasser eingewogen und gelöst (Vergleichsbeispiel)
4. Precursorlösung in Wasser verdünnt aus wässriger Precursor-Stammlösung (Vergleichsbeispiel)
5. Precursorlösung in wässriger KOH eingewogen und gelöst (Vergleichsbeispiel)

Um Katalysatoren mit unterschiedlichem Goldgehalt zu erhalten, wurde jeder Ansatz mehrfach in jeweils unterschiedlichen Chlorogoldsäure-Konzentrationen angesetzt beziehungsweise verdünnt. Es sollten Goldkatalysatoren mit Metallgehalten zwischen 0,1 und 5 % hergestellt werden. Es wurde pro Ansatz jeweils 2 g Goldkatalysator hergestellt.

### Tränken der Metalloxidträger, Incipient-Wetness-Methode

Die Precursorlösungen wurden in jeweils getrennten Ansätzen bei gleichzeitig intensiver Durchmischung nach und nach zum Trägermaterial getropft. Das Ende der Zugabe ist durch einsetzende Feuchtigkeit des Trägermaterials zu erkennen, die die Sättigung des Porenvolumens und damit die Grenze der Aufnahmefähigkeit des Trägers anzeigt.

### Trocknen, Reduktion

Die getränkten Katalysatorvorläufer wurden über Nacht im Trockenschrank getrocknet (ca. 80 °C) und anschließend für 3 h bei 250 °C im Stickstoff/Wasserstoffstrom (ca. 10 % H₂) reduziert. Danach wird im Stickstoffstrom abgekühlt.

### Ergebnisse

### a) Goldgehalt

Bei allen hergestellten Goldkatalysatoren wurde zunächst der Goldgehalt mittels ICP-AES bestimmt. Es wurden Goldkatalysatoren mit Metallgehalten zwischen 0,1 und 5 % hergestellt. Die experimentell bestimmten Goldgehalte werden mit den theoretisch berechneten verglichen.

Die theoretischen Goldgehalte und tatsächlichen Goldgehalte korrelieren in allen Ansätzen ausgezeichnet. Es gelingt, das Gold verlustfrei auf den Träger aufzutragen.

### b) Partikelgröße

TEM-Aufnahmen der Goldkatalysatoren zeigen überraschenderweise Partikelgrößen von 1 bis maximal annähernd 10 nm.

### c) Reduktionstemperatur

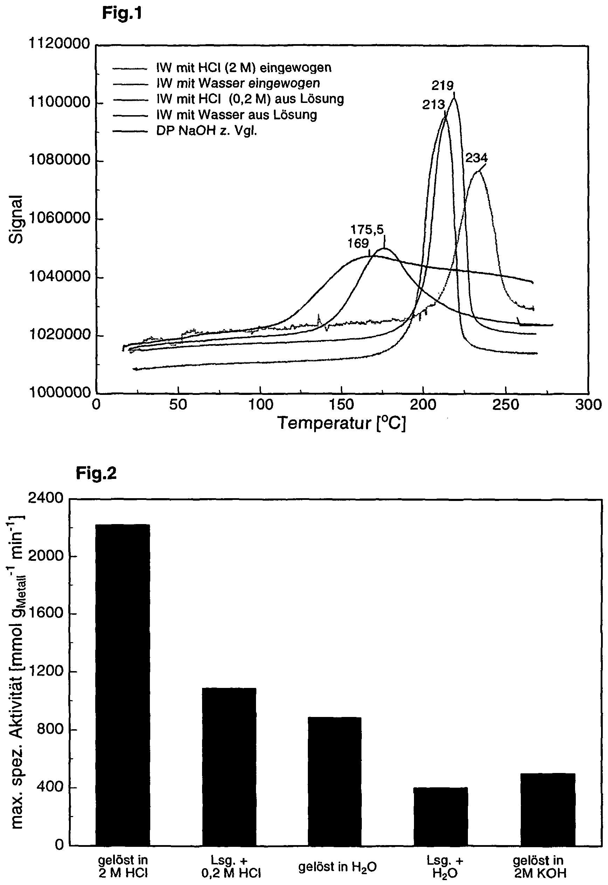

Von allen Katalysatoren wurden jeweils Profile der temperaturprogrammierten Reduktion (TPR-Profile) aufgenommen. Figur 1 zeigt die TPR-Profile der Katalysatoren. Aus den TPR-Profilen sind deutliche Unterschiede in den Maximumtemperaturen zu erkennen. Die höchste Reduktionstemperatur zeigt mit 234 °C der Katalysator, bei dem der Precursor in stark saurer Lösung (hier: 2 mol/l HCl) eingewogen wurde; die niedrigste zeigt der Katalysator, bei dem die Precursorlösung mit Wasser verdünnt wurde. Aus einer hohen Reduktionstemperatur kann auf eine starke Adsorption des Goldprecursors an den Träger geschlossen werden.

### Beispiel 2: Katalytische Oxidation von Glucose

Die katalytische Performance der nach Beispiel 1 hergestellten Katalysatoren wurde in der Flüssigphasenoxidation von Glucose zu Gluconsäure getestet.

Die Reaktion wurde in einem temperierten Glasreaktor (Volumen 500 ml) bei 40°C durchgeführt. Die Begasung erfolgte durch eine Glasfritte mit einer Sauerstoffflussrate von 500 ml/min. Die Glucoseanfangskonzentration betrug 100 mmol/l. Der pH-Wert wurde mit Hilfe eines Titrators (Titroline alpha, Fa. Schott) und 2 mol/l Kalilauge konstant bei pH 9 gehalten. Da es sich bei Gluconsäure um eine Monocarbonsäure handelt, kann bei 100 % Selektivität aus dem verbrauchten Laugenvolumen direkt auf die entstandene Säuremenge geschlossen werden. Zusätzlich erfolgte eine Kontrolle mittels HPLC.

### Ergebnisse

### a) Selektivität

Die hergestellten Goldkatalysatoren zeigen in dieser Reaktion 100% Selektivität für die Aldehydposition der Glucose.

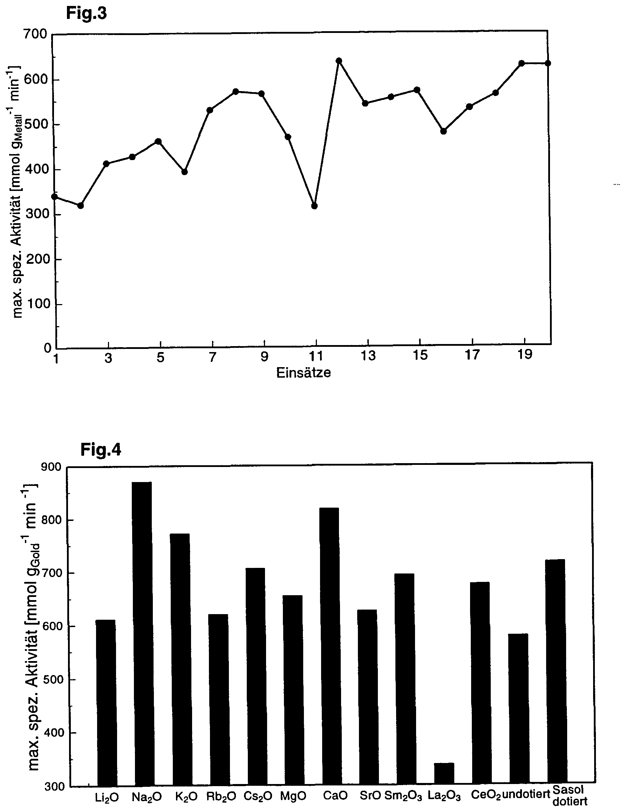

Als Vergleich diente Puralox SCFa-90 ("undotiert" in Figur 4) und das NaO-dotierte Puralox SCFa-90 ("Sasol dotiert" in Figur 4).

### b) Katalytische Aktivität

Der Umsatz war bei allen Reaktionen vollständig (100 %). Zum Vergleich der Katalysatoren wurde die maximale, spezifische Aktivität herangezogen.

Figur 2 zeigt die Abhängigkeit der Aktivität der hergestellten Katalysatoren von der verwendeten Precursorlösung. Der erfindungsgemäße Katalysator, bei dem der Precursor in stark saurer Lösung (hier: 2 mol/l HCl) gelöst wurde, zeigt überraschenderweise mit Abstand die höchste Aktivität, von 2200 mmol Glucose/g_{Metall} min.

### c) Langzeitstabilität

Ein 0,3 % Au Katalysator wurde in 20 "repeated batch"-Versuchen ohne Aktivitätsverlust eingesetzt. Bei der Untersuchung der Langzeitstabilität zeigte sich, dass die Katalysatoren eine ausgezeichnete Langzeitstabilität besitzen. Ein Goldleaching konnte nicht beobachtet werden. Die Aktivitätszunahme mit zunehmender Anzahl der Versuche ist Fall auf eine verminderte Sauerstofflimitierung durch Katalysatorverlust zurückzuführen.

### Beispiel 3: Einfluss von Promotorzusätzen

Als Promotorzusätze wurden verschiedene Alkali-, Erdalkali- und Seltenerdmetalle eingesetzt, und ihr Einfluss auf die Aktivität der nach Beispiel 1 hergestellten Goldkatalysatoren wurde untersucht.

### Ergebnisse

Figur 4 zeigt den Einfluss unterschiedlicher Promotoren auf die max. spez. Aktivität der erfindungsgemäß hergestellten Goldkatalysatoren mit 0,3 Gew.-% Gold. Der Promotorgehalt beträgt jeweils 0,1 Gew.-% am Gesamtkatalysator. Fast jeder der zugesetzten Promotoren hatte einen aktivitätssteigernden Effekt. Den größten aktivitätssteigemden Effekt hatten Natrium-, Kalium-, Caesium-, Calcium- und Ceroxide, aber auch Sm₂O₃.

### Vergleichsbeispiel: Au/TiO₂-Katalysator mit 0,5 % Au

### Herstellung

Als Trägermaterial wurde ein Anatas-haltiges TiO₂-Hydra (Kronos, S_{BET} = 288 m²/g) eingesetzt. Zu einer wässrigen Suspension von 50 g TiO₂ in einem Liter destilliertem Wasser, die auf 70°C erwärmt und mit 0,2 N NaOH auf einen pH-Wert von 6,5 eingestellt wurde, werden bei konstantem pH-Wert unter intensivem Rühren innerhalb von 3 h 500 mg Tetrachlorogoldsäure (HAuCl₄ x 3 H₂O) in 250 ml Wasser zugetropft. Der Ansatz wird bei 70°C eine Stunde weiter gerührt. Nach Abkühlung auf Raumtemperatur wird eine Magnesiumcitrat-Lösung (2,318 g MgHC₆H₅O₇ x 5 H₂O in 50 ml Wasser), deren pH-Wert zuvor mit 0,2 N NaOH auf 6,5 eingestellt wurde, zugegeben. Nach einer 1-stündigen Rührzeit wird der Feststoff abzentrifugiert, dreimal mit Wasser gewaschen und anschließend im Vakuumtrockenschrank bei einem Druck von <50 hPa 17 h bei Raumtemperatur und 4 h bei 50°C getrocknet. Der erhaltene Precursor wird leicht vermörsert und in Luft mit einer Aufheizrate von 1 K/min auf 250°C erhitzt und 3 h bei dieser Temperatur aktiviert.
Ausbeute: 47,3 g
TEM: dominierend sind Teilchen mit d kleiner als 5 nm, vereinzelte Teilchen mit d von etwa 20 nm
ICP-OES-Analyse: 0,45 %

### Glucose-Oxidation

Der 0,5 % Au-enthaltende Katalysator mit TiO₂-Träger (0,5 % Au/TiO₂) Typ 149 (ACA) wurde für die Glucose-Oxidation eingesetzt. Die Glucose-Oxidation erfolgte unter folgenden Reaktionsbedingungen: Reaktionsvolumen (batch): 500 ml, Katalysatormenge: 1 g/l, Substratanfangskonzentration: 100 mmol/l, pH-Wert:9; Temperatur: 40°C, Druck: 1 bar,O₂-Begasungsrate: 500 ml/min, Rührgeschwindigkeit: 700 UpM.

### Ergebnisse

| | |
|---|---|
| Glucoseumsatz: | 100% |
| maximale spezifische Aktivität | 416 mmol Glucose g Metall⁻¹ min⁻¹ |
| Selektivität zu Gluconsäure | >99,5% |

## Patentansprüche

1. Verfahren zur Herstellung eines geträgerten Goldkatalysators zur Oxidation von Kohlenhydraten aus porösem Metalloxidträger und Chlorogoldsäure-Precursor, enthaltend die Schritte:
a) Bereitstellen des Trägers in trockener Form,
b) Inkontaktbringen des Trägers mit einer Lösung des Chlorogoldsäure-Precursors, wobei das Volumen der Lösung maximal so groß ist wie das Porenvolumen des Trägers, so dass ein imprägnierter Katalysatorvorläufer erhalten wird, und
c) Trocknen des imprägnierten Katalysatorvorläufers,
wobei die Lösung des Chlorogoldsäure-Precursors eine Lösung von HAuCl₄ in wässriger Salzsäure mit einer Konzentration von 0,1 mol/l bis 12 mol/l Säure ist.

2. Verfahren nach Anspruch 1, wobei in Schritt b) dazu die Lösung des Chlorogoldsäure-Precursors schrittweise und nur so weit zu dem trockenen Träger gegeben wird bis der Träger kein weiteres Volumen der Lösung mehr aufnehmen kann.

3. Verfahren nach einem der Ansprüche 1 oder 2, wobei in einem weiteren Schritt d) eine Reduktion der Katalysatorvorläufer im Wasserstoffstrom bei Temperaturen von größer oder gleich 250 °C durchgeführt wird.

4. Verfahren nach Anspruch 3, wobei in Schritt d) die Reduktion für 10 min bis 300 min erfolgt.

5. Verfahren nach Anspruch 3 oder 4, wobei in Schritt d) der Wasserstoffstrom einen Wasserstoffgehalt von 5 Vol.-% bis 15 Vol.-% und gegebenenfalls Inertgas enthält.

6. Verfahren nach einem der Ansprüche 1 oder 2, wobei in einem weiteren Schritt d) eine Reduktion der Katalysatorvorläufer als Flüssigphasenreduktion durchgeführt wird.

7. Verfahren nach einem der vorstehenden Ansprüche, wobei in Schritt c) das Trocknen bei Temperaturen von 60 °C bis 200° C erfolgt.

8. Verfahren nach einem der vorstehenden Ansprüche, wobei der Metalloxidträger ausgewählt ist aus Aluminiumoxid, Siliziumoxid und Titandioxid.

9. Verfahren nach einem der vorstehenden Ansprüche, wobei zu dem Träger und/oder der Precursor-Lösung zusätzlich Dotierungszusätze, ausgewählt aus Oxiden der Alkalimetalle, Erdalkalimetalle und Seltenerdmetalle gegeben werden.

10. Verwendung einer Lösung von HAuCl₄ in wässriger Salzsäure einer Konzentration von 0,1 mol/l bis 12 mol/l Säure zur Herstellung eines Metalloxid-geträgerten Goldkatalysators für die selektive Oxidation von Kohlenhydraten durch Incipient-Wetness-Imprägnierung eines porösen Metalloxidträgers mit der Precursorlösung.

11. Metalloxid-geträgerter Goldkatalysator für die selektive Oxidation von Kohlenhydraten, herstellbar nach dem Verfahren nach einem der Ansprüche 1 bis 9.

12. Katalysator nach Anspruch 11, **dadurch gekennzeichnet, dass** die mittlere Größe der Goldpartikel auf dem Träger weniger als 10 nm beträgt.

13. Katalysator nach Anspruch 11 oder 12, **dadurch gekennzeichnet, dass** der Goldanteil von 0,01 Gew.-% bis 10 Gew.-% beträgt.

14. Verwendung des Katalysators nach einem der Ansprüche 11 bis 13 zur Oxidation von organischen Verbindungen, ausgewählt aus Alkoholen, Aldehyden und Polyhydroxyverbindungen.

15. Verwendung nach Anspruch 14, zur selektiven Oxidation von Kohlenhydraten, ausgewählt aus Glucose, Galactose, Mannose, Xylose und Ribose; Maltose, Laktose, Cellobiose und Isomaltose; Palatinose; Stärkesirupen; Maltodextrinen sowie Gemischen davon.

16. Verwendung nach Anspruch 14 zur Herstellung von Aldonsäuren.

## Claims

1. A method for producing a supported gold catalyst for the oxidation of carbohydrates from a porous metal oxide support and a chloroauric acid precursor, comprising the following steps:
a) preparing the support in dried form,
b) contacting the support with a solution of the chloroauric acid precursor, wherein the maximum volume of the solution is as great as the pore volume of the support, so that an impregnated catalyst precursor is obtained, and
c) drying the impregnated catalyst precursor,
wherein the solution of the chloroauric acid precursor is a solution of HAuCl₄ in aqueous hydrochloric acid at a concentration of 0.1 mol/l to 12 mol/l acid.

2. The method according to claim 1, wherein in step b) thereto the solution of the chloroauric acid precursor is added to the dry support gradually, and only until the support can no longer absorb an additional volume of solution.

3. The method according to any one of claims 1 or 2, wherein in an additional step d), a reduction of the catalyst precursors is performed in the hydrogen stream, at temperatures of greater than or equal to 250 °C.

4. The method according to claim 3, wherein in step d) the reduction occurs for 10 min to 300 min.

5. The method according to claim 3 or 4, wherein in step d) the hydrogen stream contains a hydrogen content of 5 vol. % to 15 vol. % and optionally contains inert gas.

6. The method according to any one of claims 1 or 2, wherein in a further step d) a reduction of the catalyst precursors as a liquid phase reduction is performed.

7. The method according to any one of the preceding claims, wherein in step c) drying takes place at temperatures of 60 °C to 200 °C.

8. The method according to any one of the preceding claims, wherein the metal oxide support is selected from aluminum oxide, silicon oxide, and titanium dioxide.

9. The method according to any one of the preceding claims, wherein doping agents selected from oxides of the alkali metals, alkaline earth metals, and rare earth metals are added to the support and/or the precursor solution.

10. Use of a solution of HAuCl₄ in aqueous hydrochloric acid at a concentration of 0.1 mol/l to 12 mol/l acid for producing a metal oxide supported gold catalyst for the selective oxidation of carbohydrates by an incipient-wetness-impregnation of a porous metal oxide support with the precursor solution.

11. A metal oxide supported gold catalyst for the selective oxidation of carbohydrates, which can be produced according to the method of any one of claims 1 to 9.

12. The catalyst according to claim 11, **characterized in that** the average size of the gold particles on the support is less than 10 nm.

13. The catalyst according to claim 11 or 12, **characterized in that** the proportion of gold amounts to 0.01 wt. % to 10 wt. %.

14. The use of the catalyst according to any one of claims 11 to 13 for the oxidation of organic compounds, selected from alcohols, aldehydes, and polyhydroxy compounds.

15. The use according to claim 14 for selective oxidation of carbohydrates, selected from glucose, galactose, mannose, xylose and ribose; maltose, lactose, cellobiose and isomaltose; palatinose; starch syrups; maltodextrins, and mixtures thereof.

16. The use according to claim 14 for producing aldonic acids.

## Revendications

1. Procédé pour la fabrication d'un catalyseur d'or supporté, pour l'oxydation des glucides, le catalyseur comportant un support poreux en oxyde métallique et un précurseur de l'acide chloraurique, le procédé contenant les étapes suivantes :
a) fournir le support sous forme sèche,
b) contacter le support avec une solution du précurseur de l'acide chloraurique, le volume de la solution étant au maximum aussi grand que le volume des pores du support, afin d'obtenir un précurseur de catalyseur imprégné, et
c) sécher le précurseur de catalyseur imprégné,
la solution du précurseur de l'acide chloraurique étant une solution de HAuCl₄ dans l'acide chlorhydrique aqueux avec une concentration de 0,1 mol/l à 12 mol/l de l'acide.

2. Procédé selon la revendication 1, dans lequel, dans l'étape b), la solution du précurseur de l'acide chloraurique est ajoutée au support sèche progressivement et jusqu'à ce que le support ne puisse plus absorber aucun volume de la solution.

3. Procédé selon l'une quelconque des revendications 1 ou 2, dans lequel, dans une autre étape d), une réduction des précurseurs de catalyseur est effectuée dans un courant d'hydrogène à des températures de 250 °C ou supérieures.

4. Procédé selon la revendication 3, dans lequel la réduction dans l'étape d) est effectuée pour 10 min à 300 min.

5. Procédé selon la revendication 3 ou 4, dans lequel le courant d'hydrogène dans l'étape d) a une teneur en hydrogène de 5 % en volume à 15 % en volume, et le cas échéant du gaz inerte.

6. Procédé selon l'une quelconque des revendications 1 ou 2, dans lequel, dans une autre étape d), une réduction des précurseurs de catalyseur est effectuée sous forme d'une réduction dans la phase liquide.

7. Procédé selon l'une quelconque des revendications précédentes, dans lequel le séchage dans l'étape c) s'effectue à des températures de 60 °C à 200 °C.

8. Procédé selon l'une quelconque des revendications précédentes, dans lequel le support en oxyde métallique est choisi parmi l'oxyde d'aluminium, l'oxyde de silicium et le dioxyde de titane.

9. Procédé selon l'une quelconque des revendications précédentes, dans lequel on ajoute en outre des additifs de dopage au support et/ou à la solution de précurseur, les additifs étant choisis parmi les oxydes des métaux alcalins, des métaux alcalino-terreux et des terres rares.

10. Utilisation d'une solution de HAuCl₄ dans l'acide chlorhydrique aqueux avec une concentration de 0,1 mol/l à 12 mol/I de l'acide, pour la fabrication d'un catalyseur d'or supporte sur un oxyde métallique, pour l'oxydation sélective des glucides, par l'imprégnation à humidification initiale d'un support poreux en oxyde métallique avec la solution de précurseur.

11. Catalyseur d'or supporté sur un oxyde métallique pour l'oxydation sélective des glucides qui peut être fabriqué selon le procédé selon l'une quelconque des revendications 1 à 9.

12. Catalyseur selon la revendication 11, **caractérisé en ce que** la taille moyenne des particules d'or sur le support est inférieure à 10 nm.

13. Catalyseur selon la revendication 11 ou 12, **caractérisé en ce que** la proportion d'or est de 0,01 % en poids à 10 % en poids.

14. Utilisation du catalyseur selon l'une quelconque des revendications 11 à 13 pour l'oxydation des composés organiques choisis parmi les alcools, les aldéhydes et des composés polyhydroxy.

15. Utilisation selon la revendication 14 pour l'oxydation sélective des glucides choisis parmi glucose, galactose, mannose, xylose et ribose ; maltose, lactose, cellobiose et isomaltose ; palatinose ; les siropes d'amidon ; maltodextrines et les mélanges de ceux-ci.

16. Utilisation selon la revendication 14 pour la fabrication des acides aldoniques.
